# EUROPÄISCHE PATENTSCHRIFT

(11) **EP 3 297 937 B1**
(45) Veröffentlichungstag und Bekanntmachung des Hinweises auf die Patenterteilung: **19.08.2020**
(21) Anmeldenummer: 16716186.8
(22) Anmeldetag: 05.04.2016
(51) Int. Cl.: B65D 90/02, B65D 90/06, C12M 1/00, C12M 1/107

(54) **GROßBEHÄLTER**
LARGE CONTAINER
GRAND RÉCEPTACLE

(30) Priorität: 19.05.2015 DE 202015102563 U
(43) Veröffentlichungstag der Anmeldung: 28.03.2018
(73) Patentinhaber: PlanET Biogas Group GmbH, 48691 Vreden (DE)
(72) Erfinder: MEYER ZU STROHE, Jörg, 46342 Velen (DE)
(74) Vertreter: Habbel, Ludwig
(86) Internationale Anmeldenummer: PCT/EP2016/057458
(87) Internationale Veröffentlichungsnummer: WO 2016/184600

(56) Entgegenhaltungen:
- EP-A1- 0 637 553
- DE-A1- 10 034 100
- DE-B- 1 287 301
- DE-B3-102007 017 206
- DE-B3-102007 017 206
- FR-A1- 2 821 105
- JP-A- S61 273 381
- JP-A- S61 273 381
- US-A- 4 625 478
- US-A- 4 625 478
- US-A- 4 625 478

## Beschreibung

Die Erfindung betrifft einen Großbehälter nach dem Oberbegriff des Anspruchs 1. Als Großbehälter werden im Rahmen des vorliegenden Vorschlags Behälter bezeichnet, die begehbar sind. Beispielsweise werden solche Großbehälter in der Landwirtschaft verwendet und beispielsweise als Fermenter im Bereich der Biogaserzeugung eingesetzt.

Aus der US 4 625 478 A, die den am nächsten kommenden Stand der Technik darstellt, ist ein Großbehälter in Form eines Turmsilos bekannt, das mittels einer für Luft undurchlässigen Auskleidung als Futtersilo mit reduzierter Sauerstoffatmosphäre ausgestaltet ist. Einzelne Folienstreifen bilden die luftundurchlässige Auskleidung und sind mit Hilfe von Schrauben, welche die Wand des Silos durchsetzen, an der Wand gehalten.

Aus der DE 100 34 100 A1 ist ein Großbehälter bekannt, der beispielsweise als Tank für Heiz- und Dieselöl, Benzin sowie Säuren, Laugen und Lösungsmittel genutzt werden kann.

Ein Großbehälter, der ebenfalls zur Aufbewahrung von Flüssigkeiten, Schüttgut und dergl. vorgesehen ist, ist aus der DE 86 33 074 U1 bekannt. In der Praxis weisen derartige Großbehälter emaillierte Stahlplatten auf, bei denen die Emaillierung den Schutz des Stahlmaterials gegenüber einem chemischen Angriff des im Großbehälter befindlichen Materials sicherstellen soll. Die einzelnen Platten müssen gegeneinander abgedichtet werden. Aus diesem Grund ist die Montage des Großbehälters vergleichsweise aufwendig. Zudem ist je nach dem verwendeten Dichtungsmaterial die Montage witterungsabhängig und dementsprechend nur bei bestimmten äußeren Witterungsbedingungen überhaupt möglich. Die Schutzschicht der Stahlplatten ist empfindlich gegenüber mechanischen Belastungen, was sowohl während der Montage als auch im späteren Betrieb zu beachten ist.

Alternativ dazu sind aus der Praxis gattungsgemäße Großbehälter bekannt, bei denen die Wand jeweils nicht emaillierte Stahlplatten aufweist, sondern Edelstahlplatten, so dass die chemische Beständigkeit der Platten auch ohne die mechanisch empfindliche emaillierte Schicht gewährleistet werden kann. Abgesehen von den vergleichsweise hohen Materialkosten derartiger Edelstahlplatten besteht auch bei diesen Großbehältern die oben erläuterte Problematik der Abdichtung zwischen den einzelnen Platten.

Aus der Praxis sind weiterhin gattungsgemäße Großbehälter bekannt, deren Wand ebenfalls aus Beton besteht. Eine gleichmäßige Qualität wird dadurch sichergestellt, dass die Wand Betonplatten aufweist, die als Fertigelemente unabhängig von der jeweiligen Witterung werkseitig vorgefertigt worden sind. Die Abdichtung der einzelnen Fertigelemente gegeneinander und die vergleichsweise hohen Transportkosten beeinflussen die Herstellung dieser Großbehälter nachteilig. Dies gilt insbesondere, wenn die Transportwege vom Hersteller zum Aufstellungsort nicht nur zig Kilometer oder wenige 100 km betragen, sondern wenn Behälter eines europäischen Herstellers auf anderen Kontinenten wie z. B. Australien oder Nordamerika errichtet werden sollen. Die FR 2 821 105 A1 oder die DE 1 287 301 B zeigen solche Behälter, deren Wände jeweils mehrere mineralische Wandsegmente aufweisen. Auch die US 4 625 478 A zeigt einen solchen Behälter, dessen Wand in einer Ausgestaltung aus mehreren Betonsegmenten aufgebaut ist.

Weiterhin sind aus der Praxis gattungsfremde Großbehälter bekannt, bei denen zumindest die Wand, ggf. jedoch Wand und Boden monolithisch aus Ortbeton hergestellt sind. Auch die bereits genannte US 4 625 478 A zeigt einen solchen Behälter, dessen Wand in einer alternativen Ausgestaltung aus Beton aufgebaut ist. Die Verfügbarkeit derartiger Großbehälter hängt unmittelbar von der Verfügbarkeit der erforderlichen Schalung ab, zudem beeinflussen auch die Transportkosten der Schalung die Kosten für die Herstellung des Behälters in einem erheblichen Umfang. Zudem ist nicht auszuschließen, dass die Qualität der fertiggestellten Großbehälter Schwankungen unterworfen ist, die durch die Sorgfalt des Montagepersonals, die Verfügbarkeit bzw. Qualität der jeweils verwendeten Zuschlagsstoffe und deren mengenmäßige Zusammensetzung beeinflussbar ist.

Aus der JP S 61 273 381 A ist eine Montagekonstruktion für eine wasserdichte Folie eines wärmegedämmten Tanks bekannt, der beispielsweise als ein Wärmespeichertank, ein Becken, ein Abwassertank, ein Entwässerungstank, ein Hauswassertank oder ein Chemietank ausgestaltet sein kann. Dabei wird ein Wärmedämmstoff auf die Oberfläche eines Betongehäuses gelegt und eine wasserdichte Folie auf das Wärmedämmstoffmaterial aufgetragen. Eine wasserdichte Folie wird auf das Wärmedämmstoffmaterial aufgetragen. Eine Sitzplatte wird hergestellt, in der das gleiche Material - hier als Befestigungsplatte bezeichnet - wie die wasserdichte Folie auf ein Harzbasismaterial laminiert ist. Die Sitzplatte wird ortsfest fixiert, indem die Befestigungsplatte, die Sitzplatte und die wasserdichte Folie durch Wärmefusion miteinander verbunden werden.

Der Erfindung liegt die Aufgabe zugrunde, einen gattungsgemäßen Großbehälter dahingehend zu verbessern, dass dieser möglichst schnell, unkompliziert und witterungsunabhängig montiert werden kann, für vielfältige Anwendungen geeignet ist, möglichst wirtschaftlich hergestellt und montiert werden kann, und einen möglichst problemlosen Betrieb des Großbehälters unterstützt. Diese Aufgabe wird gelöst durch einen Großbehälter mit den Merkmalen des Anspruchs 1. Vorteilhafte Ausgestaltungen sind in den Unteransprüchen beschrieben.

Die Erfindung schlägt mit anderen Worten vor, erstens den Großbehälter thermisch zu isolieren, indem sowohl zwischen der Folie und dem Boden als auch zwischen der Folie und den Platten der Behälterwand jeweils eine thermisch isolierende Zwischenschicht angeordnet ist, und zweitens seine innere Folienauskleidung an mehreren Stellen zu fixieren. Dadurch kann ein erfindungsgemäßer Großbehälter nicht nur als Aufbewahrungsbehälter wie ein Silo oder ein Tank genutzt werden, sondern auch als Fermenter einer Biogasanlage eingesetzt werden.

Aufgrund der thermischen Isolierung kann ein für einen Fermenter vorteilhaftes Temperaturniveau im Inneren des Großbehälters gehalten werden. Und mechanische Belastungen, die auf die innen angebrachte Folie einwirken, führen nicht zu deren Faltenbildung oder zum Ablösen von den Platten der Behälterwand, denn erfindungsgemäß ist vorgesehen, dass die einzelnen Abschnitte der verwendeten Folie ortsfest fixiert sind. So wird nicht etwa lediglich ein frei beweglicher "Foliensack" geschaffen, der lose in die Wand des Behälters eingehängt ist.

Auf diese Weise ist ausgeschlossen, dass Strömungsbewegungen innerhalb des Großbehälters zu Verschiebungen oder Verformungen der Folie führen können. Somit ist ausgeschlossen, dass die Folie beispielsweise aufgrund lokaler Überlastung einreißt und / oder in eine Ablauföffnung hinein gerät und diese verstopft, oder dass die Folie in Kollision mit Rührgeräten oder anderen Einbau-Elementen innerhalb des Großbehälters gerät.

Um sowohl die thermische Isolierschicht als auch die Folie an den Platten der Behälterwand festzulegen und insbesondere dabei sicherzustellen, dass die Schutzwirkung der Folie nicht durch die erforderlichen Halteelemente gefährdet wird, ist erfindungsgemäß folgende Konstruktion vorgesehen:
- Schrauben erstrecken sich von außen durch die Platten der Wand nach innen zu Halteblechen,
- die Haltebleche halten die thermisch isolierende Zwischenschicht an den metallischen Platten ortsfest in Position,
- außerdem legen die Haltebleche Folienstreifen fest, die zwischen der thermisch isolierenden Zwischenschicht und der Folie angeordnet sind,
- und schließlich ragen die Folienstreifen über die Haltebleche hinaus und sind im Bereich dieses Überstandes mit der Folie verschweißt.

Die Erfindung schlägt vor, den Schutz der Wand gegenüber dem im Großbehälter aufzunehmendem Material, insbesondere hinsichtlich chemischer Angriffe, nicht durch die Ausgestaltung der Platten selbst zu bewirken, sondern vielmehr dadurch, dass die Wand innen mit einer Folie ausgekleidet ist. Die Platten müssen daher lediglich eine ausreichende mechanische Stabilität aufweisen sowie eine Beständigkeit gegenüber Witterungseinflüssen. Die erheblich größeren Belastungen hinsichtlich ihrer chemischen Beständigkeit, nämlich gegenüber dem im Behälter aufzunehmenden Material, und auch eine ausreichende Dichtheit zwischen den einzelnen benachbarten Platten ist nicht erforderlich, da diese Anforderungen durch die verwendete Folie erfüllt werden, z. B. eine Kunststofffolie. Die Folie ist flüssigkeitsdicht ausgestaltet, so dass in dem Großbehälter Flüssigkeiten sicher gelagert werden können und die Platten zuverlässig geschützt sind. Vorteilhaft ist die Folie jedoch sogar gasdicht ausgestaltet, so dass aus der Flüssigkeit aufsteigende Gase ebenfalls die Folie nicht durchdringen und die Platten schädigen können.

Bei einer ersten Ausführungsform eines erfindungsgemäßen Großbehälters ist vorgesehen, dass einzelne Wandabschnitte werkseitig als Sandwichbauteile bereitgestellt werden, wobei jede Platte werkseitig mit einer Folie auf ihrer Innenseite versehen wird. Diese Folie wird auch als Folienabschnitt oder Folienstück bezeichnet, nämlich als einzelnes Element, wobei die insgesamt die Wand innen auskleidende Folie aus einer Vielzahl derartiger Folienabschnitte bzw. Folienstücke gebildet wird. Die Ausgestaltung der Platten ermöglicht einen hohen Grad an Vorfertigung des Großbehälters unabhängig von den klimatischen Bedingungen am Aufstellungsort. Bei der Herstellung des Großbehälters müssen lediglich die einzelnen Folienabschnitte der einzelnen Platten abdichtend miteinander verbunden werden, um die gesamte Folie zu schaffen. Im Vergleich zur Verwendung einer beispielsweise pastösen Dichtungsmasse, die zur Abdichtung einzelner Stahlplatten verwendet wird, ist z. B. eine Verschweißung der Folienabschnitte weniger empfindlich gegenüber unterschiedlichen Witterungseinflüssen. Bei dieser ersten Ausführungsform sind die einzelnen Folienabschnitte Teil des jeweiligen mehrschichtig aufgebauten Wandabschnitts, so dass sie ortsfest fixiert sind.

Bei einer zweiten Ausführungsform ist die Montage der Behälterwand an Ort und Stelle vorgesehen, ohne dass dazu die Verwendung von vorkonfektionierten, mehrschichtigen Wandelementen erforderlich ist. Vielmehr kann aus mehreren mechanisch stabilen Platten zunächst eine die Statik der Wand bestimmende äußere Schicht der Wand errichtet werden, und anschließend werden im Inneren dieser Behälterwand Folienabschnitte angebracht und miteinander abdichtend verbunden, beispielsweise verschweißt. Eine Fixierung dieser Folienabschnitte erfolgt mithilfe von Folienstreifen, die ihrerseits mechanisch an den stabilen äußeren Platten der Wand gehalten werden, mithilfe von Schrauben und Halteblechen. Die mechanischen Befestigungsmittel sichern einen zuverlässigen, ortsfesten Halt der Folienstreifen an den äußeren, stabilen Platten des Wandaufbaus und können zur Lastverteilung großflächig ausgestaltet sein, um zu verhindern, dass die Folienstreifen unter solchen Belastungen einreißen, die eine Relativbewegung zwischen den Folienstreifen und den Befestigungsmitteln zu bewirken bestrebt sind.

Auch wenn die mechanischen Befestigungsmittel aus diesem Grunde großflächig den Folienstreifen anliegen, ist sichergestellt, dass die Folienstreifen nicht vollflächig durch die Befestigungsmittel abgedeckt werden. Beispielsweise kann vorgesehen sein, dass die mechanischen Befestigungsmittel Aussparungen aufweisen, beispielsweise in Form von sogenannten Fenstern oder Bohrungen, durch welche ein Kontakt mit dem jeweiligen Folienstreifen möglich ist. Die Folienstreifen weisen größere Abmessungen auf als die erwähnten Haltebleche, so dass die Folienstreifen über ihre mechanischen Befestigungsmittel hinausragen. Nachdem die Folienstreifen montiert sind, wird die innere Folie montiert. Wo die Folienstreifen anschließend noch zugänglich sind, dort, wo sie über ihre mechanischen Befestigungsmittel hinausragen, wird die innere Folie mit diesen Folienstreifen verschweißt, so dass die Folie verschiebesicher an der Wand des Großbehälters festgelegt ist.

Zur Erzielung der Dichtnähte können benachbarte Folienabschnitte beispielsweise kalt oder unter Hitzeeinwirkung miteinander verschweißt werden, oder verklebt werden, oder mittels Klemmleisten dicht aneinandergepresst werden, oder auf eine andere, an sich bekannte Weise miteinander verbunden werden. Rein beispielhaft wird nachfolgend stets von einer Schweißverbindung ausgegangen, die sich in der Praxis auch für andere Folienanwendungen als Verbindung bewährt hat.

Zudem kann die Arbeit des Abdichtens unabhängig von der Errichtung der Behälterstruktur durchgeführt werden, so dass der Großbehälter zunächst errichtet werden kann und die Abdichtung, nämlich die Verschweißung der Folienabschnitte, beispielsweise später, bei geeigneter äußerer Witterung, erfolgen kann. Weiterhin ist die Folie auf der geschützten Innenseite der Wandangeordnet. Dies bedeutet, dass die Verschweißung der Folienabschnitte im trockenen und ggf. beheizbaren Innenraum des Großbehälters unabhängig von den äußeren Witterungsbedingungen erfolgen kann, falls auch bei ungünstiger äußerer Witterung die Abdichtung des Großbehälters nicht später vorgenommen werden soll, sondern eine möglichst baldige Inbetriebnahme des Großbehälters gewünscht ist.

Durch die erfindungsgemäße Ausgestaltung, insbesondere den hervorragenden Schutz der die mechanische Stabilität bewirkenden Platten gegenüber chemischen Angriffen wird der Großbehälter verwendbar als Fermenter im Bereich der Biogaserzeugung, aber auch beispielsweise zum Lagern von Rübenmus, welches mit einem pH-Wert von etwa 2,5 so hohe Anforderungen an die chemische Beständigkeit der Wand des Großbehälters stellt, dass selbst Platten aus einem vergleichsweise teureren Material wie einem V2A-Edelstahl für derartige Anwendungsfälle nicht geeignet sind. Zudem stellt die Folie eine optimale Abdichtung sicher, so dass beispielsweise der Großbehälter gasdicht ausgestaltet werden kann, wie dies z. B. bei einem Fermenter oder einem Gärrestlager im Bereich der Biogaserzeugung erforderlich ist.

Es kann beispielsweise vorgesehen sein, die aneinander angrenzenden Folienstücke mittelbar miteinander zu verschweißen, indem ein Folienstreifen auf die Fuge zwischen den beiden angrenzenden Folienstücken aufgelegt und mit beiden Folienstücken verschweißt wird. In diesem Fall können die die einzelnen Abschnitte der insgesamt die Innenauskleidung des Großbehälters bewirkenden Folie so angeordnet werden, dass sie einander nicht notwendigerweise überlappen müssen. Beispielsweise können die Folienstücke der oben erwähnten ersten Ausführungsform dieselben Abmessungen aufweisen wie die jeweilige Platte von hoher mechanischer Stabilität, an der ein solches Folienstück befestigt ist, oder sie können sogar etwas geringere Abmessungen aufweisen als die jeweilige Platte.

Ein erster besonders vorteilhafter Aspekt der erfindungsgemäßen Konstruktion liegt darin, dass die sich zur Schaffung einer Dichtnaht überlappenden Folienabschnitte bzw. Folienstreifen oder Folienstücke zwischen sich eine Dichtebene aufweisen, die bei einem kreisrunden Behälter parallel zur Wandfläche, also tangential verläuft. Im Behälter herrschender Innendruck, z. B. aufgrund des Eigengewichts einer Flüssigkeit, drückt die sich überlappenden Flächen zweier Folienabschnitte bzw. eines Folienstreifens und eines Folienabschnitts aneinander und verbessert so die Dichtwirkung der Dichtnaht. Im Unterschied dazu werden dickwandige Platten, z. B. in Form von Betonfertigteilen, auf Stoß nebeneinander montiert. Die Dichtebene zwischen zwei benachbarten Platten verläuft daher radial, durch den Mittelpunkt des Behälters, und der im Behälter herrschende Innendruck ist bestrebt, die beiden Platten auseinander zu bewegen. Dieser Innendruck ist somit bestrebt, die Dichtwirkung nicht zu verstärken, sondern zu schwächen.

Vorteilhaft kann jedoch anstelle der oben erläuterten mittelbaren Verbindung benachbarter Folienabschnitte vorgesehen sein, dass die Folienabschnitte einander überlappen und unmittelbar miteinander verbunden sind. Hierzu können die einzelnen Folienabschnitte bei der ersten Ausführungsform, welche die vorkonfektionierten, mehrschichtigen Wandelemente betrifft, jeweils größer bemessen sind als die jeweilige Platte, so dass die Folienabschnitte an einem oder an mehreren Rändern der Platte über die Platte hinausragen und benachbarte Folienabschnitte einander überlappen. Bei einer rechteckigen Platte kann beispielsweise vorgesehen sein, dass die Folie an zwei benachbarten der vier Ränder über die Platte übersteht, so dass insgesamt ein etwa dachziegelartig überlappender Verbund der Folienstücke bei einer Vielzahl von die Wand bildenden Platten ermöglicht wird. Bei der zweiten Ausführungsform können die einzelnen Folienabschnitte beispielsweise als lange Bahnen ausgestaltet sein, welche sich jeweils über die gesamte Höhe der Wand des Großbehälters erstrecken. Durch die überlappende Ausgestaltung der Folienabschnitte wird die Herstellung des Behälters vereinfacht, indem nämlich nicht, wie weiter oben erwähnt, ein zusätzlicher Folienstreifen gehandhabt zu werden braucht. Zudem stellt eine einzige Schweißnaht die dichte Verbindung zweier benachbarter Folienabschnitte sicher, während ein zusätzlich aufgebrachter Folienstreifen zwei derart dichte Schweißnähte erfordern würde, nämlich mit jedem der beiden zu überbrückenden Folienabschnitte.

Dabei kann grundsätzlich vorgesehen sein, zusätzliches Material zwischen den beiden einander überlappenden Folienabschnitten anzuordnen, beispielsweise einen Zwischenstreifen aus einem anderen Material als die beiden Folienabschnitte, wobei dieser Zwischenstreifen seinerseits mit jedem der beiden Folienabschnitte besser verschweißbar ist als die beiden Folienabschnitte miteinander. Vorteilhaft jedoch kann vorgesehen sein, die beiden Folienabschnitte unmittelbar miteinander zu verschweißen, so dass die beiden oben erwähnten Vorteile der Überlappung besonders stark zur Geltung kommen. So ist es beispielsweise nicht erforderlich, einen Zwischenstreifen oder dergleichen zu handhaben und zwischen die beiden einander überlappenden Folienbereiche einzubringen, und auch in radialer Richtung, also quer zur Dichtebene, wird auf diese Weise nur eine einzige Schweißnaht geschaffen, und nicht zwei Schweißnähte, mit denen der erwähnte Zwischenstreifen ansonsten in radialer Richtung einerseits an die innere Folie und andererseits an die äußere Folie anschließen würde.

Vorteilhaft können die Platten der Wand jeweils Abmessungen aufweisen, die größer sind als die in der Praxis üblichen Abmessungen von maximal 3 m Länge und maximal 1,25 m Breite. Insbesondere können die Platten vorteilhaft Abmessungen von etwa 2m x 6m aufweisen. Im Vergleich zu den praxisüblichen Platten, die beispielsweise 1,25m x 2m groß sind, wird so ein deutlich schnellerer Montagefortschritt ermöglicht und eine Materialeinsparung hinsichtlich der erforderlichen Verbindungselemente wie Niete oder Schrauben ermöglicht. Insbesondere das als vorteilhaft angegebene Maß von 2 x 6 m ermöglicht einerseits den noch problemlosen Transport der einzelnen Platten auf LKWs oder in Containern und reduziert andererseits die Anzahl der zu verarbeitenden Platten mit den entsprechenden vorteilhaften Auswirkungen auf die Anzahl der Verbindungselemente der Platten. Bei der ersten Ausführungsform, bei welcher jede Platte mit einem eigenen Folienabschnitt werkseitig vorkonfektioniert ist, wirkt sich Größe der Platten auf die Länge der insgesamt herzustellenden Schweißnähte aus, welche für die Abdichtung, also für die Verbindung der einzelnen benachbarten Folienabschnitte miteinander, erforderlich ist. Demgegenüber kann bei der zweiten Ausführungsform die Gesamtlänge der herzustellenden Dichtungsnähte nochmals kürzer ausfallen, da einzelne Folienbahnen sich über mehrere Platten hinweg erstrecken können.

Die Begrenzung auf vergleichsweise kleine Abmessungen der Platte von z. B. den genannten 1,25m x 2m ist durch unterschiedliche Einflüsse bedingt, von denen sich der vorliegende Vorschlag frei macht: emaillierte Platten müssen davor geschützt werden, dass die Emaillierung während der Handhabung der Platten abplatzt. Überschreiten die Platten bestimmte Abmessungen, so besteht die Gefahr, dass sie sich während des Transports und während der Montage so stark durchbiegen, dass Risse in der vergleichsweise spröden Emailschicht entstehen. Zudem weisen große Platten ein so hohes Gewicht auf, dass sie nur mithilfe von Hebewerkzeugen gehandhabt werden können. Haken oder ähnliche Einrichtungen der Hebewerkzeuge bergen die Gefahr, an den Platten Abplatzer der Emaillierung zu verursachen. Die vergleichsweise kleineren Plattenmaße vermeiden beide Probleme: erstens biegen sich die Platten aufgrund des geringeren Eigengewichts nicht unschädlich weit durch, so dass Risse vermieden werden können. Zweitens ermöglicht dieses geringere Eigengewicht, dass die Platten von Hand, ohne die Verwendung von Hebewerkzeugen, gehandhabt werden können, so dass durch die entsprechend schonende Handhabung die Abplatzer vermieden werden können. Der vorliegende Vorschlag geht von der Überlegung aus, dass diese tradierten Plattenabmessungen und die damit verbundenen Nachteile überwunden werden können, so dass durch größere Plattenabmessungen die genannten Vorteile eines schnelleren Montagefortschritts und einer geringeren Gesamtlänge der herzustellenden Dichtungsnähte erzielt werden können.

Vorteilhaft können die für die Wand verwendeten Platten aus Metall, und insbesondere vorteilhaft aus Stahl bestehen. Durch die vergleichsweise geringen möglichen Wandstärken kann im Vergleich zu Betonsegmenten erhebliches Gewicht eingespart werden, so dass problemlos ein wirtschaftlicher Transport der Wandplatten von einem Herstellungswerk zu dem jeweiligen Aufstellungsort möglich ist. Durch die vergleichsweise erheblich geringere Wandstärke ist auch das erforderliche Transportvolumen geringer. Die mechanische Stabilität der metallischen Platten, insbesondere wenn sie als Stahlplatten ausgestaltet sind, ist trotz der geringen Wandstärke für die angegebenen Verwendungszwecke gut geeignet.

Je nach dem verwendeten Material kann es wirtschaftlich vorteilhaft sein, statt eines korrosionsbeständigen Metalls ein Metall zu verwenden, welches eine zusätzliche Behandlung in Form eines Korrosionsschutzes erfordert. In an sich bekannter Weise können die Stahlplatten problemlos und auf wirtschaftliche Weise gegen Witterungseinflüsse geschützt werden, beispielsweise vorteilhaft mittels einer Lackierung, wobei vorteilhaft praktisch sämtliche Farbtöne beispielsweise der RAL-Farbpalette zur Verfügung stehen, so dass je nach Wunsch des Herstellers oder des Betreibers des Großbehälters die nach außen sichtbare Farbgebung der Wand gestaltet werden kann. Ein mehrlagiger Aufbau der Beschichtung kann vorteilhaft vorgesehen sein, um einen besonders zuverlässigen Witterungs- bzw. Korrosionsschutz und / oder eine möglichst vielseitige optische äußere Gestaltung der Platten zu ermöglichen. Beispielsweise kann eine erste, unterste bzw. innere Lage der Beschichtung in Form einer so genannten kathodischen Tauchbad-Lackierung bzw. KTL-Beschichtung vorgesehen sein, oder eine äußere Lage der Beschichtung kann beispielsweise in Form einer Pulverlackierung vorgesehen sein.

In einer Ausgestaltung des Großbehälters, die nicht als erfindungsgemäß angesehen wird, kann vorgesehen sein, dass die Wand an eine beispielsweise aus Ortbeton bereitgestellte Bodenplatte angeflanscht wird, und das die Folie und / oder der Flansch mittels einer Dichtungsmasse gegen über der Bodenplatte abgedichtet wird.

Erfindungsgemäß ist vorgesehen, dass die Folie auch den Boden auskleidet, beispielsweise wenn eine besonders hohe chemische Beständigkeit des Bodens gegenüber dem im Großbehälter aufzunehmendem Material gewünscht ist. Die Folienauskleidung des Großbehälters kann in diesem Fall topfartig ausgestaltet werden, indem eine entsprechende Bodenfolie mit der Wandfolie verschweißt wird, so dass eine Abdichtung des gesamten Wand- und Bodenbereichs sichergestellt werden kann. Diese Ausgestaltung des Großbehälters bedeutet, dass der Boden keine besonders hohe chemische Beständigkeit aufweisen muss, sondern dass er in erster Linie lediglich die erforderliche Tragfähigkeit aufweisen muss. Beispielsweise kann in einer ersten Variante die Wand als mehrteiliger Stahlring einfach auf den - üblicherweise zuvor planieren - Erdboden gestellt werden. Anschließend wird die Folie in diesen Stahlring eingebracht, so dass das Behälterinnere gegenüber dem Boden und der Wand abgedichtet ist. In einer vorteilhaften zweiten Variante kann vorgesehen sein, dass der Boden durch eine Schicht Ortbeton gebildet ist, wobei die Qualität des Betonbodens nicht hinsichtlich ihrer Beständigkeit an das im Behälter aufzunehmende Material angepasst zu sein braucht. Vielmehr kann es in vielen Fällen ausreichend sein, dass diese Betonschicht einen ausreichend sicheren Verbissschutz bildet, um eine Beschädigung der Folie z. B. durch Nagetiere zu verhindern.

Durch den erfindungsgemäßen Aufbau der Wand des Großbehälters wird der Vorteil erzielt, dass die Folie im Großbehälter fixiert ist, da die Folie mithilfe von mechanischen Befestigungsmitteln an der Platte fixiert ist. Zudem kann auch vorteilhaft vorgesehen sein, dass die Bodenfolie über die Fläche des Bodens verteilt an mehreren Stellen am Boden festgelegt sein, beispielsweise mittels einer Technik, wie sie aus dem Bereich der Flachdach-Abdichtung für die Festlegung der Dachfolien an sich bekannt ist. Durch die Fixierung der Folie wird eine Verwendung des Großbehälters ermöglicht, bei welcher ein Rührwerk in dessen Inneren vorgesehen ist oder bei welcher auf andere Weise, z. B durch das Strömungsverhalten einströmender oder ausströmender Materialien, das im Großbehälter befindliche Material eine erhebliche Bewegungsenergie auf den Boden bzw. die Wand zu übertragen bestrebt ist. Durch die Festlegung der Folie ist sichergestellt, dass sich die Folie nicht von der Wand bzw. vom Boden löst und beispielsweise in das erwähnte Rührwerk gerät. Im Vergleich dazu, die Folie einfach in Art eines Sackes in den Großbehälter einzuhängen wird die mechanische Belastung der Folie möglichst gering gehalten wird und beispielsweise ein Ausreißen der Folie bzw. deren Ablösung von den Platten der Wand bzw. vom Boden vermieden, was ansonsten die Dichtheit des Großbehälters gefährden würde.

Erfindungsgemäß ist eine thermisch isolierende Zwischenschicht zwischen der Folie und den Platten der Wand und zwischen der Folie und dem Boden vorgesehen. Beispielsweise bei der Verwendung des Großbehälters als Fermenter zur Biogaserzeugung kann es vorteilhaft sein, ein bestimmtes Temperaturniveau im Großbehälter sicherzustellen, so dass mittels der thermisch isolierenden Zwischenschicht Temperaturverluste vermieden werden können. Die Zwischenschicht kann - insbesondere bei der ersten Ausführungsform mit sandwichartigen Aufbau - bereits werksseitig vormontiert werden, beispielsweise an den Platten befestigt werden, so dass auch bei Verwendung einer thermisch isolierenden Zwischenschicht die Montage des Großbehälters unkompliziert und in kurzer Zeit erfolgen kann.

Die Verwendung von zwei oder mehr Zwischenschichten anstelle einer einzigen, dementsprechend dickeren Zwischenschicht kann vorteilhaft sein, wenn statt eines textilen, zum Beispiel vliesartigen Materials ein vergleichsweise härteres Material verwendet wird, beispielsweise EPS-Platten. In diesem Fall ist nämlich bei der Verwendung von EPS-Platten mit großer Wandstärke nicht auszuschließen, dass bei Biegung der EPS-Platten Falten oder Knicke auftreten, so dass eine vollflächige Anlage der Isolierschicht-Platten an der z. B. aus Stahl bestehenden Platte nicht gewährleistet werden kann und die Gefahr von Wärmebrücken bzw. Kältebrücken besteht, welche die Wirksamkeit der angestrebten thermischen Isolierung beeinträchtigen. Die äußeren, z. B. stählernen Platten verlaufen entsprechend dem gewünschten Durchmesser des Großbehälters gebogen. Entsprechend gebogen verlaufende, an den jeweiligen Radius angepasste Isolierkörper zu verwenden, würde der erläuterten Gefahr begegnen, wäre jedoch wirtschaftlich nachteilig. Die Verwendung von zwei oder mehr Zwischenschichten ermöglicht eine faltenfreie Anlage der jeweiligen Zwischenschicht an einer benachbarten Zwischenschicht bzw. an der äußeren, stählernen Platte unter Verwendung wirtschaftlicher, nämlich plan hergestellter Zwischenschichtplatten.

Um Korrosionsprobleme an der Innenseite der z. B. aus Stahl bestehenden äußeren Platten zuverlässig zu vermeiden, und auch um einen unerwünschten Feuchtigkeitseintrag in eine thermisch isolierende Schicht zu vermeiden, kann vorteilhaft eine Belüftung an der Innenseite der äußeren Platten vorgesehen sein. Zu diesem Zweck kann vorteilhaft eine so genannte Distanzbahn der Platte innen anliegend angeordnet sein, wobei ein an sich bekanntes Material verwendet werden kann, um die Distanzbahn zu bilden, beispielsweise ein Vlies, ein Abstandsgewebe, eine Noppenfolie oder dergleichen.

Dabei ist wesentlich, dass durch die Distanzbahn ein Konvektionsraum geschaffen wird, welcher eine Belüftung der Platten an deren Behälterinnenseite ermöglicht. Falls sich Kondenswasser an dieser Innenseite in größeren Mengen bilden sollte, kann dies einerseits problemlos durch den Konvektionsraum nach unten abfließen, oder es kann andererseits - bei geringeren Mengen - aufgrund der durch den Konvektionsraum strömenden Belüftung problemlos verdunsten.

Die Verwendung der Distanzbahn bietet zudem die Möglichkeit, den Großbehälter als doppelwandigen Behälter auszugestalten, ohne hierfür sämtliche äußeren - z. B. metallischen - Platten gegeneinander abdichten zu müssen. In diesem Fall ist dann vorgesehen, dass die Distanzbahn eine Schicht aufweist, die für das im Großbehälter zu lagernde Fluid dicht ist. Bei Verwendung einer Noppenfolie als Distanzbahn ist diese Fluiddichtheit ohnehin gegeben, da die gesamte Noppenfolie aus einer solchen, dreidimensional verformten, fluiddichten Schicht besteht. Während die innere Folie des Großbehälters die erste dichte Behälterwand bildet, können die fluiddichten Schichten benachbarter Distanzbahnen - beispielsweise zwei benachbarte Noppenfolien - miteinander dicht verbunden werden, beispielsweise verklebt oder verschweißt werden, und somit eine zweite Dichtebene bilden.

Ein Ausführungsbeispiel eines erfindungsgemäßen Großbehälters wird anhand der rein schematischen Darstellungen nachfolgend näher erläutert. Dabei zeigt
- Fig. 1: einen vertikalen, teilweise weggebrochenen Schnitt durch den Wandbereich des Großbehälters, und
- Fig. 2: einen vertikalen Schnitt durch einen anderen Ausschnitt des Wandbereichs, in einem gegenüber Fig. 1 größeren Maßstab.

In den Zeichnungen ist jeweils mit 1 insgesamt ein Großbehälter angedeutet. Dieser weist eine aus Ortbeton hergestellte Bodenplatte 2 auf. Ein erstes, unteres Wandsegment einer Wand 3 ist auf die Bodenplatte 2 aufgeflanscht, an der Außenseite mittels eines L-förmigen Bodenflansches 4, und an der Innenseite mittels eines flach auf der Bodenplatte 2 aufliegenden Innenflansches 5.

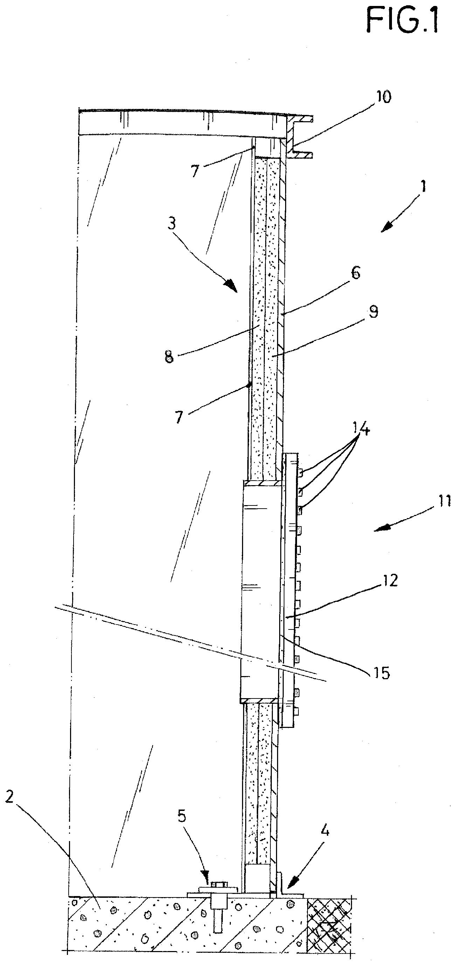

Die Wand 3 wird insgesamt durch eine Vielzahl von Wandsegmenten gebildet. Jedes Wandsegment weist eine Platte 6 auf, die aus Stahl besteht und gegen Witterungseinflüsse mittels einer Lackierung, beispielsweise in Form einer Pulverlackierung, geschützt ist. Die Platte 6 ist auf der Innenseite mit einer Folie 7 versehen, die aus einem chemisch in hohem Maße beständigen Kunststoff besteht, der beispielsweise eine hohe Beständigkeit gegen Säuren aufweist. Die Folie 7 ist nicht unmittelbar an der Platte 6 befestigt, sondern an einer inneren thermisch isolierenden Zwischenschicht 8, welche ihrerseits bei dem Ausführungsbeispiel der Fig. 1 mit einer äußeren thermisch isolierenden Zwischenschicht 9 verbunden ist, die an der Platte 6 festgelegt ist, beispielsweise mit der Platte 6 verklebt ist. Die Zwischenschichten 8 und 9 bestehen aus expandiertem Polystyrol und können sowohl von der Schichtdicke als auch vom Raumgewicht und ihren übrigen Materialeigenschaften identisch sein.

Bei dem Ausführungsbeispiel der Fig. 1 stehen sowohl die Folie 7 auf der Innenseite als auch die stählerne Platte 6 auf der Außenseite nach oben über die Zwischenschichten 8 und 9 vor. Ein zweites Wandsegment kann also nach oben an das in der Zeichnung dargestellte Wandsegment anschließen, wobei die Folie 7 des unteren Wandsegments die Folie 7 des oberen Wandsegments überlappt, so dass in diesem Überlappungsbereich eine dichte Folienschweißnaht hergestellt werden kann.

Ein U-förmiger Verbindungsflansch 10 verläuft entlang dem oberen Rand der Platte 6 und ist mit der Platte 6 fest verbunden, beispielsweise verschweißt oder verschraubt. Der Verbindungsflansch 10 erstreckt sich über die Platte 6 hinaus nach oben, so dass er in gleicher Weise mit dem unteren Rand des oben anschließenden benachbarten Wandsegments verbunden werden kann.

Rein schematisch ist bei dem in Fig. 1 dargestellten Wandsegment angedeutet, dass eine Wartungsöffnung 11 vorgesehen sein kann. Diese ist mittels einer Verschlussplatte 12 verschlossen, wobei die Verschlussplatte 12 mittels einer Vielzahl von Verschraubungen 14 mit der Platte 6 fest, aber lösbar verbunden ist. Um die Dichtheit der Wartungsöffnung 11 zu gewährleisten, ist zwischen der Verschlussplatte 12 und der Platte 6 eine Dichtung 15 vorgesehen. Diese Dichtung 15 kann aus demselben Material wie die Folie 7 bestehen, sie kann jedoch auch durch eine pastöse Dichtungsmasse gebildet sein, die nach jeder Demontage der Verschlussplatte 12 und vor der erneuten Montage dieser Verschlussplatte 12 neu aufgetragen wird, oder sie kann als mehrfach wiederverwendbare Dichtung 15 beispielsweise in Form einer Moosgummidichtung ausgestaltet sein.

Die beschriebene Abdichtung der Wartungsöffnung 11 dient in erster Linie dazu, einen Schutz gegen von außen eindringende Feuchtigkeit zu schaffen, z. B. aufgrund von Niederschlägen. Die Abdichtung zum Behälterinneren hin erfolgt im Bereich der Folie 7. Hierzu kann vorgesehen sein, dass sich ein Rohrstutzen von der Wartungsöffnung 11 ins Innere des Großbehälters 1 erstreckt, wobei beidseitig von der Folie 7 jeweils ein Dichtungsring aus einem Elastomerwerkstoff angeordnet ist und diese Dichtungsanordnung als Pressringdichtung bzw. Ringraumdichtung ausgestaltet ist die in axialer Richtung verpresst werden kann und sich dabei in radialer Dichtung ausdehnt, so dass sie dem Rohrstutzen abdichtend anliegt.

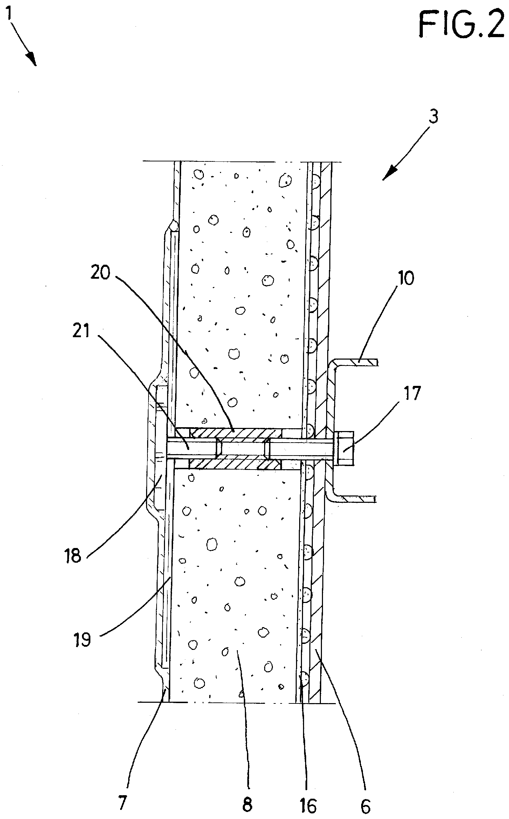

In der Fig. 2 ist abweichend von der Fig. 1 rein beispielhaft lediglich eine einzige thermisch isolierende Zwischenschicht 8 vorgesehen, die aus einem geschäumten Material besteht. Jedoch ist auch bei der Fig. 2 zusätzlich zu der thermisch isolierenden Zwischenschicht 8 eine zweite Schicht zwischen der äußeren, metallischen Platte 6 und der inneren Folie 7 angeordnet: eine Noppenfolie 16, welche eine Basisfläche aufweist sowie Noppen, die sich von der Basisfläche weg erstrecken, grenzt an die metallische Platte 6. Die Noppen sind zur Platte 6 hin ausgerichtet und dienen auf diese Weise die Noppen als Abstandshalter. Sie schaffen eine an die Platte 6 grenzende Konvektionsebene, die zur Belüftung und somit als Korrosionsschutz für die metallischen Platten 6 der Wand 3 dient.

Angesichts der Abmessungen des Großbehälters 1 werden mehrere als Bahnen, Platten oder ähnlich bezeichnete separate Abschnitte von Noppenfolien 16 verwendet. Diese sind, bezogen auf das in dem Großbehälter 1 zu lagernde Fluid, miteinander fluiddicht verschweißt. Der Großbehälter 1 gemäß Fig. 2 erfüllt daher besonders hohe Sicherheitsanforderungen, da er als doppelwandiger Behälter ausgestaltet ist und zwei separate Dichtebenen aufweist, die nämlich durch die miteinander verschweißten Abschnitte der Folie 7 einerseits und durch die miteinander verschweißten Noppenfolien 16 geschaffen werden.

Auch in der Fig. 2 ist die Folie 7 an der Wand 3 hinsichtlich ihrer Position zu den metallischen Platten 6 fixiert: dabei erstrecken sich Schrauben 17 im Bereich der U-Profile 10 von außen nach innen zu Halteblechen 18. Die Haltebleche 18 halten nicht nur die Noppenfolien 16 und die innere Zwischenschicht 8 an den metallischen Platten 6 ortsfest in Position, sondern legen auch Folienstreifen 19 fest, die zwischen der Zwischenschicht 8 und der Folie 7 angeordnet sind. Die Folienstreifen 19 ragen über die Haltebleche 18 hinaus und sind im Bereich dieses Überstandes mit der Folie 7 verschweißt, so dass auf diese Weise die Folie 7 an den metallischen Platten 6 verschiebesicher fixiert ist.

An den Halteblechen 18 sind Gewindehülsen 20 festgelegt, welche jeweils eine Schraube 17 aufnehmen. Die Gewindehülsen 20 ermöglichen durch ihre jeweilige Länge einen dementsprechenden Längenausgleich der Verschraubung, beispielsweise um Toleranzen in der sich insgesamt ergebenden Wandstärke der aus mehreren Schichten bestehenden Wand 3 auszugleichen. Die Gewindehülsen 20 sind an beiden axialen Enden offen und auf Halteschrauben 21 aufgeschraubt, welche ihrerseits an den Halteblechen 18 befestigt sind, beispielsweise mit einem Halteblech 18 verschraubt, verklebt oder verschweißt sind.

Die Ausgestaltung gemäß der Fig. 2 ermöglicht einen Aufbau der Wand 3 an Ort und Stelle, ohne mehrere Schichten aufweisende, vorkonfektionierte Wandabschnitte. Vielmehr können die metallischen Platten 6 zu einer metallischen Wand errichtet werden, und anschließend kann von innen die Montage und Verschweißung der Noppenfolien 16 erfolgen und die Montage der thermisch isolierenden Zwischenschicht 8. Dieser Wandaufbau wird anschließend gesichert durch das Einbringen der Schrauben 17 und der Haltebleche 18. Schließlich werden die einzelnen Abschnitte der Folie 7 im Behälterinneren angebracht, indem beispielsweise entsprechend lange Abschnitte der Folie 7 vom oberen Rand der Wand 3 nach unten bis zum Boden der Wand 3 abgerollt werden. Die Abschnitte der Folie 7 werden an den über die Haltebleche 18 überstehenden Bereichen der Folienstreifen 19 durch Verschweißung festgelegt und zudem an ihren benachbarten und mit einander überlappenden Rändern miteinander verschweißt.

## Patentansprüche

1. Großbehälter (1),
mit einem Boden
und einer geschlossen umlaufenden Wand (3),
wobei die Wand (3) eine Vielzahl einzelner, miteinander verbundener Platten (6) aufweist, die Platten (6) an ihrer ins Behälterinnere gerichteten Innenseite jeweils mit einer Folie (7) versehen sind,
wobei die aneinander grenzenden, benachbarten Folien (7) durch Dichtnähte flüssigkeitsdicht miteinander verbunden sind,
wobei die Folie (7) an der Wand (3) hinsichtlich ihrer Position zu den metallischen Platten (6) durch Schrauben (17) fixiert ist,
**dadurch gekennzeichnet,**
**dass** auch der Boden mit einer Folie (7) ausgekleidet ist, zwischen der Folie (7) und dem Boden eine thermisch isolierende Zwischenschicht (8) angeordnet ist,
und auch zwischen der Folie (7) und der jeweiligen Platte (6) eine thermisch isolierende Zwischenschicht (8, 9) angeordnet ist,
und **dass** sich die Schrauben (17) im Bereich von U-Profilen (10) von außen durch die Platten (6) nach innen zu Halteblechen (18) erstrecken,
wobei die U-Profile (10) jeweils entlang dem oberen Rand der Platte (6) verlaufen, mit der Platte (6) fest verbunden sind, und sich über die Platte (6) hinaus nach oben erstrecken, in der Art, dass sie in gleicher Weise mit dem unteren Rand eines oben anschließenden benachbarten Wandsegments verbindbar sind, und die Haltebleche (18) die Zwischenschicht (8) an den metallischen Platten (6) ortsfest in Position halten
und Folienstreifen (19) festlegen, die zwischen der Zwischenschicht (8) und der Folie (7) angeordnet sind, wobei die Folienstreifen (19) über die Haltebleche (18) hinausragen und im Bereich dieses Überstandes mit der Folie (7) verschweißt sind,
und wobei an beiden axialen Enden offene Gewindehülsen (20) auf Halteschrauben (21) aufgeschraubt sind, welche ihrerseits an den Halteblechen (18) befestigt sind, und die Gewindehülsen (20) jeweils eine Schraube (17) aufnehmen.

2. Großbehälter nach Anspruch 1,
**dadurch gekennzeichnet,**
**dass** jede Platte (6) als vorkonfektioniertes Wandelement mit einer eigenen Folie (7) versehen ist,
wobei die Folie (7) jeweils größer bemessen ist als die jeweilige Platte (6), derart, dass sich die Folien (7) benachbarter Platten (6) überlappen.

3. Großbehälter nach Anspruch 1,
**dadurch gekennzeichnet,**
**dass** die Folien (7) sich über mehrere Platten (6) hinweg erstrecken.

4. Großbehälter nach Anspruch 3,
**dadurch gekennzeichnet,**
**dass** die Folien (7) sich jeweils über die gesamte Höhe der Wand (3) erstrecken.

5. Großbehälter nach einem der vorhergehenden Ansprüche,
**dadurch gekennzeichnet,**
**dass** die Folien (7) einander überlappen und in ihren Überlappungsbereichen jeweils unmittelbar miteinander verschweißt sind.

6. Großbehälter nach einem der vorhergehenden Ansprüche,
**dadurch gekennzeichnet,**
**dass** die Platten (6) jeweils eine Länge von mehr als 3 m aufweisen.

7. Großbehälter nach Anspruch 6,
**dadurch gekennzeichnet,**
**dass** die Platten (6) jeweils eine Länge von 6 m aufweisen.

8. Großbehälter nach einem der vorhergehenden Ansprüche, **dadurch gekennzeichnet,**
**dass** die Platten (6) jeweils eine Breite von mehr als 1,5 m aufweisen.

9. Großbehälter nach Anspruch 8,
**dadurch gekennzeichnet,**
**dass** die Platten (6) eine Breite von 2 m aufweisen.

10. Großbehälter nach einem der vorhergehenden Ansprüche,
**dadurch gekennzeichnet,**
**dass** die Platten (6) jeweils aus Stahl bestehen.

11. Großbehälter nach einem der vorhergehenden Ansprüche,
**dadurch gekennzeichnet,**
**dass** die Platten (6) jeweils eine Lackierung als Oberflächenbeschichtung aufweisen.

12. Großbehälter nach einem der vorhergehenden Ansprüche,
**dadurch gekennzeichnet,**
**dass** die Folie (7) an mehreren über die Fläche des Bodens verteilten Stellen am Boden festgelegt ist.

13. Großbehälter nach einem der vorhergehenden Ansprüche,
**dadurch gekennzeichnet,**
**dass** zwischen der Folie (7) und der jeweiligen Platte (6) wenigstens zwei thermisch isolierende Zwischenschichten (8, 9) angeordnet sind.

14. Großbehälter nach einem der vorhergehenden Ansprüche,
**dadurch gekennzeichnet,**
**dass** an die Innenseite der Platten (6) angrenzend eine Distanzbahn angeordnet ist, welche einen belüftbaren Zwischenraum zwischen der Folie (7) und den Platten (6) schafft.

15. Großbehälter nach Anspruch 14,
**dadurch gekennzeichnet,**
**dass** die Distanzbahn eine fluiddichte Schicht aufweist, und dass die fluiddichten Schichten benachbarter Distanzbahnen fluiddicht miteinander verbunden sind, bezogen auf das in dem Großbehälter zu lagernde Fluid.

## Claims

1. Large container (1) having a base part and a side wall (3) extending all the way around without a break,
wherein the side wall (3) is made up of a large number of individual plates (6) joined together,
and the plates (6) are each provided with a foil (7) on their inner side oriented towards the inside of the container,
wherein adjacent foils (7) that adjoin one another are joined together liquid-tightly by means of sealing welds,
wherein the foil (7) is fastened in position on the side wall (3) relative to the metal plates (6) by means of bolts (17),
**characterised in that** also the base part is lined with a foil (7),
and a thermally insulating intermediate layer (8) is disposed between the foil (7) and the base part
and a thermally insulating intermediate layer (8, 9) is also disposed between each foil (7) and its plate (6),
and that the bolts (17) in the area of U-profiles (10) extend through the plates (6) from the outside to the inside into retaining plates (18),
wherein the U-profiles (10) each extend along the upper edge of a plate (6) and are fastened immovably to the plate (6) and extend upwards over the plate (6)
in such a way that the U-profiles (10) can be attached in the same way to the bottom edge of an adjacent side wall segment disposed above it
and the retaining plates (18) hold the intermediate layer (8) firmly in place on the metal plates (6) and hold in place foil strips (19) that are disposed between the intermediate layer (8) and the foil (7),
wherein the strips of foil (19) protrude above the retaining plates (18) and are welded to the foil (7) in the area of protrusion,
and wherein open threaded sleeves (20) are screwed at both axial ends onto retaining bolts (21) that in their turn are fastened to the retaining plates (18) and the threaded sleeves (20) each receive a bolt (17).

2. Large container in accordance with claim 1, **characterised in that** each plate (6) is provided with its own foil (7) to form a pre-assembled side wall element, wherein each foil (7) is fashioned larger than its plate in such a way that the foils (7) on adjacent plates (6) overlap.

3. Large container in accordance with claim 1, **characterised in that** the foils (7) extend right across a number of plates (6).

4. Large container in accordance with claim 3, **characterised in that** the foils (7) each extend the full height of the side wall (3).

5. Large container in accordance with any one of the foregoing claims, **characterised in that** the foils (7) overlap and are welded to one other direct in the areas of overlap.

6. Large container in accordance with any one of the foregoing claims, **characterised in that** the plates (6) are each more than 3 m long.

7. Large container in accordance with claim 6, **characterised in that** the plates (6) are each 6 m long.

8. Large container in accordance with any one of the foregoing claims, **characterised in that** the plates (6) are each more than 1.5 m wide.

9. Large container in accordance with claim 8, **characterised in that** the plates (6) are each 2 m wide.

10. Large container in accordance with any one of the foregoing claims, **characterised in that** the plates (6) are each made from steel.

11. Large container in accordance with any one of the foregoing claims, **characterised in that** the plates (6) are each provided with a paint coat as a surface coating.

12. Large container in accordance with any one of the foregoing claims, **characterised in that** the foil (7) is attached to the base part at a number of points distributed over the surface of the base part.

13. Large container in accordance with any one of the foregoing claims, **characterised in that** at least two thermally insulating intermediate layers (8, 9) are disposed between the foil (7) and its plate (6).

14. Large container in accordance with any one of the foregoing claims, **characterised in that** a spacing strip is disposed adjacently against the inner side of the plates (6) to create a ventilatable intermediate space between the foil (7) and the plates (6).

15. Large container in accordance with claim 14, **characterised in that** the spacing strip incorporates a liquid-tight layer and that the liquid-tight layers of adjacent spacing strips are joined together liquid-tightly in respect of the liquid to be stored in the large container.

## Revendications

1. Grande cuve (1) comprenant un fond et une paroi périphérique (3) fermée,
sachant que la paroi (3) présente un grand nombre de plaques (6) individuelles reliées entre elles,
que les plaques (6) sont respectivement munies d'un film (7) sur leur côté intérieur regardant vers l'intérieur de la cuve,
sachant que les films (7) voisins mutuellement limitrophes sont reliés entre eux, de manière étanche aux fluides, par des cordons de soudure d'étanchéité,
sachant que le film (7) contre la paroi (3) est, quant à sa position par rapport aux plaques métalliques (6), immobilisé par des vis (17),
**caractérisée en ce que** le fond est lui aussi chemisé avec un film (7),
qu'entre le film (7) et le fond est disposée une couche intermédiaire (8) thermo-isolante, et qu'aussi entre le film (7) et la plaque (6) respective est disposée une couche intermédiaire (8, 9) thermo-isolante,
et **en ce que** les vis (17) dans la zone de profilés en U (10) s'étendent de l'extérieur vers l'intérieur en direction de tôles de retenue (18) en traversant les plaques (6),
sachant que le tracé des profilés en U (10) suit le bord supérieur de la plaque (6), que les profilés sont fermement reliés à la plaque (6) et qu'ils s'étendent vers le haut au-delà de la plaque (6),
de telle sorte qu'ils sont reliables de la même manière avec le bord inférieur d'un segment pariétal voisin juxtaposé en haut,
et que les tôles de retenue (18) maintiennent sur place, en position, la couche intermédiaire (8) contre les plaques (6) et immobilisent des rubans (19) de film disposés entre la couche intermédiaire (8) et le film (7),
sachant que les rubans (19) de film font saillie au-dessus des tôles de retenue (18) et qu'ils sont soudés avec le film (7) dans la zone de ce dépassement,
et sachant qu'aux deux extrémités axiales sont vissées des douilles filetées (20) sur des vis de retenue (21), vis qui de leur côté sont fixées contre les tôles de retenue (18), et que les douilles filetées (20) reçoivent chacune une vis (17).

2. Grande cuve selon la revendication 1, **caractérisée en ce que** chaque plaque (6) en tant qu'élément pariétal prééquipé est munie de son propre film (7), sachant que le film (7) est chaque fois dimensionné plus grand que la plaque (6) respective, de sorte que les films (7) de plaques (6) voisines se chevauchent.

3. Grande cuve selon la revendication 1, **caractérisée en ce que** les films (7) s'étendent sur plusieurs plaques (6) à la fois.

4. Grande cuve selon la revendication 3, **caractérisée en ce que** les films (7) s'étendent respectivement sur toute la hauteur de la paroi (3).

5. Grande cuve selon l'une des revendications précédentes, **caractérisée en ce que** les films (7) se chevauchent et qu'ils sont directement soudés ensemble au niveau de leurs zones de chevauchement.

6. Grande cuve selon l'une des revendications précédentes, **caractérisée en ce que** les plaques (6) présentent chacune une longueur de plus de 3 m.

7. Grande cuve selon la revendication 6, **caractérisée en ce que** les plaques (6) présentent chacune une longueur de 6 m.

8. Grande cuve selon l'une des revendications précédentes, **caractérisée en ce que** les plaques (6) présentent chacune une largeur de plus de 1,5 m.

9. Grande cuve selon la revendication 8, **caractérisée en ce que** les plaques (6) présentent une largeur de 2 m.

10. Grande cuve selon l'une des revendications précédentes, **caractérisée en ce que** les plaques (6) sont chacune en acier.

11. Grande cuve selon l'une des revendications précédentes, **caractérisée en ce que** les plaques (6) présentent chacune une peinture sous forme de revêtement superficiel.

12. Grande cuve selon l'une des revendications précédentes, **caractérisée en ce que** le film (7) est immobilisé sur la surface du fond en plusieurs endroits répartis sur le fond.

13. Grande cuve selon l'une des revendications précédentes, **caractérisée en ce qu**'entre le film (7) et la plaque (6) respective sont disposées au moins deux couches intermédiaires (8, 9) thermo-isolantes.

14. Grande cuve selon l'une des revendications précédentes, **caractérisée en ce que** contre le côté intérieur des plaques (6) est disposée de manière limitrophe une feuille d'écartement créant un espace intermédiaire ventilable entre le film (7) et les plaques (6).

15. Grande cuve selon la revendication 14, **caractérisée en ce que** la feuille d'écartement présente une couche étanche aux fluides et **en ce que** les couches étanches aux fluides de feuilles d'écartement voisines sont reliées étanches aux fluides entre elles, par rapport au fluide à stocker dans la grande cuve.
